(19) Europäisches Patentamt
European Patent Office
Office européen des brevets

(11) **EP 2 991 773 B1**

(12) **FASCICULE DE BREVET EUROPEEN**

(45) Date de publication et mention de la délivrance du brevet:
**07.03.2018 Bulletin 2018/10**

(21) Numéro de dépôt: **14727597.8**

(22) Date de dépôt: **29.04.2014**

(51) Int Cl.:
***B05B 17/06*** (2006.01)    ***B60H 1/32*** (2006.01)
***F24F 6/14*** (2006.01)    ***A61L 9/14*** (2006.01)
***B05B 7/00*** (2006.01)    ***A47F 3/00*** (2006.01)
*B05B 12/08* (2006.01)    *B64D 13/06* (2006.01)
*F24F 6/00* (2006.01)

(86) Numéro de dépôt international:
**PCT/FR2014/051025**

(87) Numéro de publication internationale:
**WO 2014/177805 (06.11.2014 Gazette 2014/45)**

(54) **SYSTEME DE NEBULISATION POUR RAFRAICHIR L'AIR**

VERNEBELUNGSVORRICHTUNG ZUM KÜHLEN VON LUFT

NEBULIZER FOR COOLING THE AIR

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **30.04.2013 FR 1353966**

(43) Date de publication de la demande:
**09.03.2016 Bulletin 2016/10**

(73) Titulaire: **ARECO FINANCES ET TECHNOLOGIE - ARFITEC**
**06130 Grasse (FR)**

(72) Inventeurs:
• **GSCHWIND, Michel**
**F-06130 PLACASSIER (FR)**
• **RICHARD, Frédéric**
**F-06220 Vallauris (FR)**
• **SABRAOUI, Abbas**
**F- 06130 GRASSE (FR)**

(74) Mandataire: **Schmidt, Martin Peter**
**IXAS Conseil**
**15, rue Emile Zola**
**69002 Lyon (FR)**

(56) Documents cités:
**EP-A1- 0 691 162    EP-A1- 0 931 595**
**FR-A1- 2 716 415    FR-A1- 2 787 352**
**FR-A1- 2 788 706**

**Description**

**Domaine technique de l'invention**

L'invention concerne les systèmes de nébulisation aptes à générer un brouillard de micro-gouttelettes d'un liquide, par exemple d'eau, dans le but de rafraîchir l'atmosphère, et plus particulièrement les systèmes de nébulisation de petite taille qui peuvent être montés sur un étal de vente pour humidifier et rafraîchir des produits frais exposés à la vente, ou encore dans un véhicule pour humidifier et rafraîchir l'air et le rendre agréable à respirer.

**Etat de la technique**

De tels systèmes sont connus en tant que tels. Le brevet EP 0 691 162 décrit un système de nébulisation avec une buse de concentration dans laquelle un élément piézoélectrique immergé dans l'eau génère un brouillard de gouttelettes d'eau à la sortie d'une buse qui concentre les ultrasons générés par ledit élément piézo-électrique en son point de sortie ; le brouillard est ensuite emporté par un courant d'air généré par un ventilateur. Cette buse est en règle générale disposée verticalement, avec la sortie focalisante pointant vers le haut ; la buse peut aussi être inclinée, par exemple à 45°.

De tels systèmes sont utilisés couramment sur des étals de vente de produits frais ; cela correspond normalement à un environnement stationnaire et stable. En revanche, aucune utilisation n'est connue dans des véhicules, qui représentent un environnement non stationnaire et perturbé. Par ailleurs, les systèmes utilisés sur des étals sont perfectibles dans la mesure où un étal, lui aussi, peut être perturbé par des chocs et d'autres perturbations mécaniques, dans la mesure où il est entouré par des personnes qui peuvent entrer en contact mécanique avec lui.

Plus particulièrement, les perturbations mécaniques peuvent engendrer une fluctuation de l'alimentation en eau de la buse de concentration. Or, si l'élément piézoélectrique n'est pas constamment immergé pendant son fonctionnement il peut être endommagé.

La demanderesse s'est aperçu que les mesures constructives qui visent à réduire l'encombrement du système, et en particulier sa hauteur, tendent à augmenter le risque que l'élément piézo-électrique se trouve temporairement incomplètement immergé ou à même sec. Plus particulièrement, on observe que lorsque l'on cherche à incliner la buse, ce qui contribue à diminuer la hauteur totale du système, le fonctionnement du système résiste moins bien à des perturbations mécaniques que dans le cas d'une buse verticale. De même, lorsque l'on cherche à diminuer la quantité globale d'eau dans le système, ce qui contribue à diminuer l'encombrement total du système, on augmente le risque d'un manque d'eau dans la buse.

**Objet de l'invention**

La présente invention constitue une amélioration de ce système, notamment en vue de son utilisation dans des environnements perturbés par le mouvement, l'accélération, la vibration ou le choc, et notamment dans des véhicules.

Plus précisément, le but de l'invention est de présenter un dispositif de nébulisation compact, robuste, fiable et simple à utiliser, léger et bon marché, qui peut être utilisé pour humidifier, rafraîchir et/ou parfumer et/ou désinfecter l'air de l'habitacle d'un véhicule.

Un autre but est de présenter un dispositif de nébulisation compact, robuste, fiable et simple à utiliser, léger et bon marché, qui peut être utilisé pour humidifier ou rafraîchir des marchandises, notamment des produits frais, exposés à la vente sur un étal. Encore un autre but est de présenter un dispositif de nébulisation compact, robuste, fiable et simple à utiliser, léger et bon marché, qui peut être utilisé pour humidifier, rafraîchir et/ou parfumer et/ou désinfecter l'air d'un local stationnaire, tel qu'un atelier, et en particulier dans des conditions mécaniquement perturbées, par exemple par des vibrations ou chocs.

L'exigence de compacité résulte du besoin d'une petite taille générale du dispositif, et en particulier d'une hauteur limitée, qui est particulièrement forte lorsque le dispositif doit être intégré dans un habitacle de véhicule.

L'exigence de robustesse résulte du besoin de résistance du dispositif contre des conditions perturbées, et de son fonctionnement fiable dans des conditions perturbées, telles que les pentes, l'accélération et le freinage brusques, les vibrations, les chocs. Elle résulte également du souhait d'éviter une maintenance fréquente du nébuliseur.

L'exigence de simplicité d'utilisation résulte notamment de l'impossibilité pratique de demander à l'utilisateur de veiller à l'approvisionnement régulier du nébuliseur en eau.

L'exigence de légèreté résulte du besoin général de limiter la masse qui s'ajoute à un véhicule (et notamment à un aéronef) par l'ajout d'options et fonctions supplémentaires.

L'exigence de prix milite en faveur d'un dispositif de construction simple.

Ces objectifs sont atteints par un dispositif de nébulisation apte à générer et à répande un brouillard de micro-gouttelettes pour rafraîchir et/ou humidifier l'atmosphère ambiante d'un local et/ou pour rafraîchir et/ou humidifier des produits exposés sur un présentoir de vente, et/ou pour rafraîchir et/ou parfumer l'atmosphère d'un habitacle, ledit dispositif comportant

(a) une buse de nébulisation pourvue d'au moins un orifice d'admission de liquide et d'au moins un orifice de sortie de liquide, et au côté opposé dudit orifice de sortie un élément piézo-électrique apte à émettre des ondes acoustiques dans ledit liquide, et la section transversale de ladite buse présentant un rétrécissement progressif en direction dudit premier orifice de sortie, de manière à ce que dans ladite buse les ondes acoustiques soient focalisées pour créer un brouillard de gouttelettes dudit liquide ;

(b) un réservoir de collecte qui alimente ladite buse en liquide,

(c) une pompe dite « pompe de circulation » reliée d'une part au réservoir de collecte et d'autre part à ladite buse par le au moins un orifice d'admission aménagé dans ladite buse, ladite pompe de circulation étant apte à générer dans ladite buse une pression de liquide suffisante pour maintenir un jet de liquide sortant par ledit orifice de sortie de la buse,

(d) une chambre de mise en pression qui est traversée par le liquide sortant de la pompe de circulation avant son entrée dans ladite buse,

ledit dispositif de nébulisation étant caractérisé en ce que le volume de la partie supérieure de la chambre de mise en pression se situant à un niveau de liquide supérieur au plus haut des trois points suivants : l'orifice d'admission d'eau de la buse situé le plus haut, le bord supérieur de l'orifice de sortie de la buse, le point le plus haut de la l'élément piézo-électrique, est au moins deux fois (de préférence au moins six fois et encore plus préférentiellement au moins douze fois) plus grand que le volume de la buse. Ce dispositif de nébulisation forme le premier objet de l'invention. Il peut être réalisé selon différents modes de réalisation et variantes.

La section d'admission de la buse (i.e. la somme des surfaces des orifices d'admission) doit être supérieure à la section de l'orifice de sortie, et de préférence au moins trois fois supérieure, afin d'éviter le phénomène de cavitation dans la buse.

Ainsi, le dispositif selon l'invention présente un rapport V5/V4 et un dimensionnement de l'orifice de sortie de la buse et des orifices d'admission de liquide de la buse tels que lors que la pompe de circulation cesse d'apporter de l'eau, l'élément piézo-électrique reste immergé dans ledit liquide (et pourrait ainsi continuer à fonctionner sans risque d'endommagement) pendant une durée $t_s$ qui est avantageusement d'au moins une seconde et de préférence comprise entre 1 et 10 secondes, et de préférence entre 2 et 5 secondes.

Grâce à sa buse à focalisation d'ondes acoustiques générées par un élément piézo-électrique, le dispositif de nébulisation selon l'invention est apte à créer et répandre un brouillard formé de gouttelettes d'un diamètre moyen typique compris entre 0,5 $\mu$m et 10 $\mu$m, de préférence entre 1 $\mu$m et 5 $\mu$m.

La focalisation des ondes acoustiques sera plus efficace pour créer un brouillard de gouttelettes dudit liquide si le rétrécissement progressif de la section transversale de ladite buse en direction dudit premier orifice de sortie est tel que les ondes acoustiques soient focalisées au niveau dudit orifice de sortie. La focalisation se fera avantageusement dans l'axe longitudinal de la buse qui traverse le centre de l'orifice de sortie, et encore plus avantageusement dans le plan horizontal dudit orifice ou légèrement à l'extérieur de ce plan.

Ledit liquide à nébuliser est préférablement de l'eau, qui peut comporter des additifs, tels que des parfums et/ou des produits désinfectants (par exemple : $H_2O_2$, acide peracétique, acide citrique).

Le dispositif selon l'invention comprend avantageusement des moyens de ventilation pour créer un flux d'air qui emporte ledit brouillard de gouttelettes vers l'extérieur dudit dispositif.

D'une manière générale, ladite pompe de circulation peut être de tout type approprié ; une pompe hélice convient bien. Elle est avantageusement située au-dessous du réservoir de collecte.

Dans un mode de réalisation du dispositif selon l'invention, ledit réservoir de collecte comporte au moins une plaque de stabilisation du niveau de liquide, disposée horizontalement, verticalement ou en biais, comportant chacune au moins une ouverture. Cela sécurise l'alimentation de la pompe de circulation en liquide, stabilise le jet de liquide et fiabilise le fonctionnement du dispositif.

Avantageusement, lesdites plaques de stabilisation sont au nombre d'au moins deux et sont disposées de manière à ce que les ouvertures soient décalées les unes par rapport aux autres. Cela améliore leur effet de stabilisation du niveau de liquide dans le réservoir de collecte. Alternativement ou en plus, au moins une partie desdites ouvertures peuvent être obturées par un clapet qui s'ouvre au moins partiellement sous une pression d'eau venant d'un côté et se ferme sous une pression d'eau venant du côté opposé, ou s'ouvre moins largement sous une pression d'eau venant d'un côté que sous une pression d'eau équivalente venant du côté opposé.

Dans un autre mode de réalisation, au moins une desdites plaques de stabilisation est réalisée au moins partiellement sous la forme d'une grille ou passoire.

Un autre moyen pour rendre le fonctionnement du dispositif insensible contre les perturbations mécaniques consiste en une construction spécifique du fond dudit réservoir de collecte, qui est incliné en direction d'un orifice d'évacuation par lequel le liquide entre dans ladite pompe de circulation. Dans une variante, le réservoir de collecte présente une forme d'entonnoir.

Dans un mode de réalisation qui peut être combiné avec tous les autres, l'axe longitudinal de ladite buse forme un angle d'inclinaison α par rapport à l'horizontale qui se situe entre 0° et 45°, de préférence entre 0° et 30° et encore plus préférentiellement entre 5° et 20°. Cela permet une construction du dispositif particulièrement compacte.

Le dispositif peut comporter un tube de collecte apte et disposé à recueillir le jet de liquide sortant de l'orifice de sortie et à se vider dans ledit réservoir de collecte. Il peut être incliné par rapport à la verticale. Ce tube de collecte peut être traversé par ledit flux d'air, qui emporte ledit brouillard de gouttelettes vers sa sortie. Cela diminue la hauteur du dispositif et simplifie sa construction.

Dans un mode de réalisation qui peut être combiné avec les précédents, le réservoir de collecte et ladite buse forment un seul bloc.

Dans un autre mode de réalisation qui peut être combiné avec les précédents, le dispositif comprend un réservoir secondaire de liquide relié au réservoir primaire, ledit réservoir secondaire étant de préférence un réservoir souple ou semi-rigide. Il alimente, de manière permanente ou intermittente, ledit réservoir de collecte, de préférence par l'intermédiaire d'une pompe.

Dans un mode de réalisation qui peut être combiné avec le précédent ou avec tous les autres, le système de nébulisation selon l'invention est alimenté en eau par un système de récupération d'eau de provenance extérieure audit système de nébulisation. Cette eau de récupération peut être de l'eau de condensation qui se forme sur des surfaces des matériaux en contact avec l'eau fonte de glace utilisée pour la réfrigération directe des produits frais exposés sur un étal.

Le dispositif peut comporter un moyen de chauffage apte à évaporer le liquide résiduel dans ledit dispositif après son arrêt. Le même moyen de chauffage peut être utilisé pour chauffer l'eau contenue dans le dispositif à une température suffisante pour diminuer sa teneur en germes pathogènes.

Le dispositif selon l'invention peut comporter également au moins un moyen de détection d'un manque de liquide associé à une boucle de rétroaction pour couper ou diminuer l'intensité des ondes acoustiques émises par l'élément piézo-électrique en cas de manque d'eau. Ce moyen de détection peut être un capteur (par exemple un capteur de niveau d'eau dans le réservoir primaire, et/ou un capteur de pression dans la chambre de mise en pression), ou une pluralité de capteurs, et/ou peut comprendre une mesure d'un paramètre électrique de la pompe de circulation.

Un autre objet de l'invention est un procédé de mise en route d'un dispositif selon l'invention, dans lequel

(a) on fait entrer du liquide dans le réservoir primaire par l'orifice d'entrée ;

(b) lorsque le niveau dudit liquide monte dans ledit réservoir primaire jusqu'à un point préréglé qui est détecté par un détecteur de niveau d'eau dans le réservoir primaire, on met en fonctionnement la pompe de circulation ;

(c) la pompe de circulation crée une pression de liquide suffisante pour que le liquide puisse envahir la buse, éventuellement après avoir envahi la chambre de mise en pression, et pour former un jet de liquide stable qui sort de l'orifice de sortie, sachant que pendant au moins une partie de ce temps, on fait entrer du liquide dans le réservoir primaire par l'orifice d'entrée ;

(d) lorsque le niveau dudit liquide dans ledit réservoir primaire a atteint un point préréglé qui est détecté par un détecteur de niveau, on active l'alimentation électrique de l'élément piézo-électrique pour créer des gouttelettes de liquide.

Plus particulièrement, ledit procédé de mise en route peut être appliqué à un dispositif de nébulisation apte à générer et à répandre un brouillard de micro-gouttelettes pour rafraîchir et/ou humidifier l'atmosphère ambiante d'un local et/ou pour rafraîchir et/ou humidifier des produits exposés sur un présentoir de vente, et/ou pour rafraîchir et/ou parfumer l'atmosphère d'un habitacle, ledit dispositif comportant

- une buse de nébulisation pourvue d'au moins un orifice d'admission de liquide et d'au moins un orifice de sortie de liquide, et au côté opposé dudit orifice de sortie un élément piézo-électrique apte à émettre des ondes acoustiques dans ledit liquide, et la section transversale de ladite buse présentant un rétrécissement progressif en direction dudit premier orifice de sortie, de manière à ce que dans ladite buse les ondes acoustiques soient focalisées pour créer un brouillard de gouttelettes dudit liquide (sachant que dans ladite buse les ondes acoustiques pouvant être focalisés par exemple au niveau dudit orifice de sortie) ;
- un réservoir de collecte qui alimente ladite buse en liquide,
- une pompe dite « pompe de circulation » reliée d'une part au réservoir de collecte et d'autre part à ladite buse par le au moins un orifice d'admission aménagé dans ladite buse, ladite pompe de circulation étant apte à générer dans ladite buse une pression de liquide suffisante pour maintenir un jet de liquide sortant par ledit orifice de sortie de la buse,
- une chambre de mise en pression qui est traversée par le liquide sortant de la pompe de circulation avant son entrée dans ladite buse,

ledit dispositif de nébulisation étant caractérisé en ce que le volume de la partie supérieure de la chambre de mise en pression se situant à un niveau de liquide supérieur au plus haut des trois points suivants : l'orifice d'admission d'eau de la buse situé le plus haut, le bord supérieur de l'orifice de sortie de la buse, le point le plus haut de la céramique, est au moins deux fois (de préférence au moins six fois et encore plus préférentiellement au moins douze fois) plus grand que le volume de la buse.

Ce dispositif auquel est appliqué ledit procédé de mise en route peut présenter l'ensemble des variantes décrites en relation avec ce dispositif, ou seulement certaines d'entre elles.

Dans l'étape (d) dudit procédé de mise en route, ledit point préréglé et/ou ledit détecteur de niveau peuvent être le(s) même(s) qu'à l'étape (b)).

Un autre objet de l'invention est l'utilisation du dispositif de nébulisations selon l'invention pour générer et répandre un brouillard de micro-gouttelettes pour rafraîchir et/ou humidifier l'atmosphère ambiante d'un local et/ou pour rafraîchir et/ou humidifier des produits exposés sur un présentoir de vente, et/ou pour rafraîchir et/ou parfumer l'atmosphère d'un habitacle d'un véhicule, notamment d'un véhicule terrestre, maritime ou aérien.

## Figures

Le dispositif selon l'invention est illustré de manière schématique par les figures 1 à 5 qui en montrent différents modes de réalisation.

La figure 1 montre un dispositif selon l'invention, en vue latérale (figure 1a) et vu du haut (figure 1b). Les figures 2a à 2d montrent de manière schématique et successive quatre phases de la mise en route et du fonctionnement du dispositif de la figure 1.

La figure 3 montre un autre dispositif selon l'invention, en vue latérale (figure 3a) et vu du haut (figure 3b). Les figures 4a à 4d montrent manière schématique et successive quatre phases de la mise en route et du fonctionnement du dispositif de la figure 3.

La figure 5 montre un autre mode de réalisation du dispositif selon l'invention : les figures 5a et 5b montrent le même dispositif et se distinguent uniquement par les repères numériques et repères géométriques qui n'ont pas pu être portés tous sur la même figure pour une raison d'encombrement.

Liste des repères utilisés sur les figures :

| | | | |
|---|---|---|---|
| 1 | Céramique piézo-électrique | 18 | Micro-gouttelettes d'eau |
| 2 | Joint | 19 | Brouillard |
| 3 | Support de la céramique | 20 | Ouverture dans la plaque de stabilisation |
| 4 | Buse de concentration | 21 | Paroi de la buse |
| 5 | Chambre de mise en pression | 22 | Paroi de la chambre de mise en pression |
| 6 | Réservoir de collecte (primaire) | 23 | Base de la buse |
| 7 | Tube de guidage de la diffusion | 24 | Capteur de la présence d'eau |
| 8 | Conduit de remplissage chambre 5 | 25 | Orifice d'évacuation du réservoir 6 |
| 9 | Capteur de la présence d'eau | 26 | Fond du réservoir de collecte 6 |
| 10 | Pompe de circulation | 40 | Dispositif de nébulisation |
| 11 | Entrée d'air | | |
| 12 | Sortie de nébulisation | V1 | Volume d'eau dans le réservoir 6 |
| 13 | Entrée remplissage en eau | V2 | Volume d'eau dans la pompe 10 |
| 14 | Orifice d'admission d'eau de la buse | V3 | Volume d'eau dans la chambre 5 (inf) |
| 15 | Orifice de sortie de la buse | V4 | Volume d'eau dans la buse 4 |
| 16 | Plaques de stabilisation | V5 | Volume d'eau dans la chambre 5 (sup) |
| 17 | Jet d'eau | Q | Débit d'eau généré par la céramique 1 |

## Description détaillée

Le système ou dispositif **40** de nébulisation selon l'invention comprend une buse de concentration **4**, de type connu, apte à contenir un liquide à pulvériser (typiquement de l'eau) et présentant un orifice de sortie **15**, la section transversale de l'intérieur de ladite buse de concentration **4** présentant un rétrécissement progressif en direction dudit orifice de sortie **15.** Ladite buse **4** présente en outre, sur le côté opposé à son orifice de sortie **15**, un élément (céramique) piézo-électrique **1** apte à émettre des ondes acoustiques dans le liquide. La paroi interne de ladite buse **4** est en un matériau dur apte à réfléchir les ondes acoustiques générées par ledit élément piézo-électrique **1.** La forme convergente des parois internes de la buse **4** est déterminée de manière à faire focaliser les ondes acoustiques ultra-soniques à un endroit proche de la partie centrale de l'orifice de sortie **15** ; ainsi est généré un brouillard de micro-gouttelettes du liquide à pulvériser lorsque la buse **4** est remplie de liquide et la céramique **1** émet des ondes acoustiques de fréquence et intensité appropriées. Ladite forme convergente des parois internes de la buse **4** est de préférence parabolique, ce qui améliore le rendement de la buse de concentration **4.** Ladite forme des parois internes de la cuve **4** montre très avantageusement une symétrie radiale.

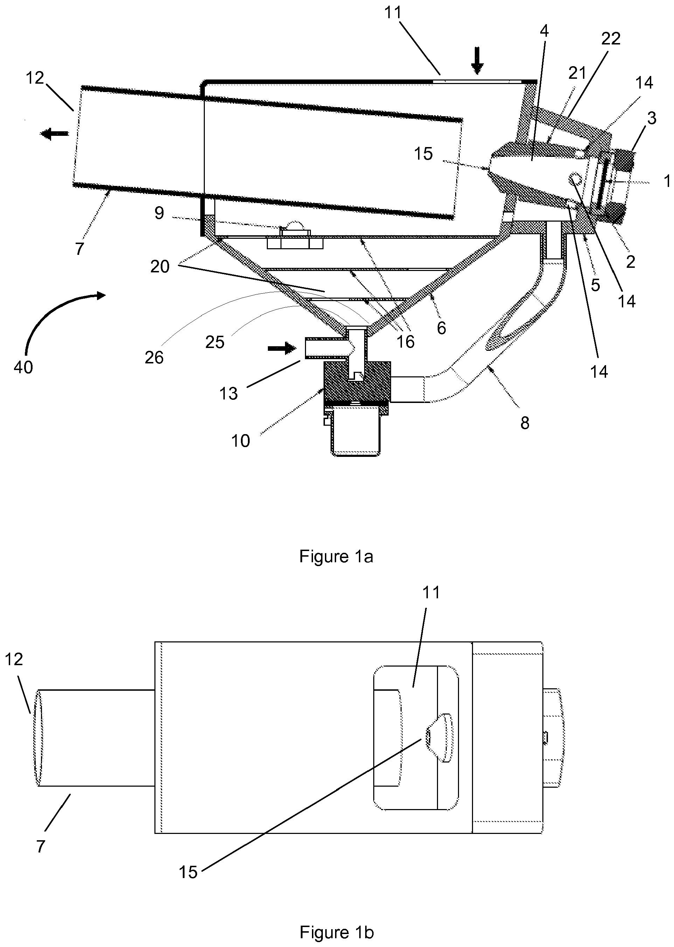

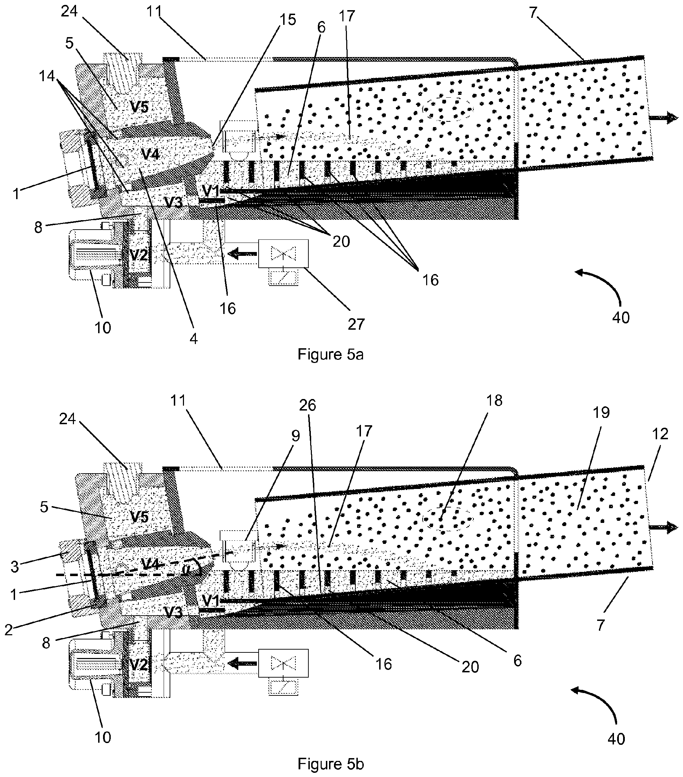

Selon l'invention, et comme illustré sur la <u>figure 1</u>, l'axe longitudinal de la buse **4** est incliné par rapport à la verticale. Cette inclinaison, exprimée par l'angle α par rapport à l'horizontale (voir la figure 5b), vise à diminuer la hauteur totale du dispositif **40.** L'angle α peut être inférieur à 65°, de préférence inférieur à 45°, plus préférentiellement inférieur à 30° et encore plus préférentiellement inférieur à 15°. Il peut être de 5° ou de 0° (axe longitudinal de la buse orientée dans le plan horizontal), et peut être même négatif, car lors du fonctionnement de l'élément piézoélectrique **1**, l'intérieur de la buse **4** est rempli du liquide à pulvériser par une pompe **10** qui maintient une pression de liquide dans ladite buse **4** ; cela nécessite une pompe **10** d'une capacité suffisante. L'inclinaison peut être de -90°, c'est-à-dire que l'orifice de sortie **15** se trouve en bas de la buse **4** ; cela donne un bon rendement de brouillard mais le système de nébulisation **40** présente alors une hauteur plus grande que lorsque l'inclinaison α est de 0° ; cette plus grande hauteur peut rendre plus difficile son intégration dans un habitacle de véhicule.

Les inventeurs ont trouvé qu'un angle α compris entre 0° et 45° (de préférence entre 0° et 30°, et encore plus préférentiellement entre 5° et 20°) assure un excellent compromis entre le rendement de nébulisation, la taille de la pompe **10** et l'encombrement du système **40** (auquel contribue la dimension de la pompe **10**). Dans le cadre de la présente invention on préfère un angle α compris entre 0 et 30°, et de préférence entre 5 et 20°.

De manière connue, la buse **4** comporte au moins un orifice d'admission de liquide **14** permettant de remplir ladite buse **4** de liquide à pulvériser. Ce remplissage a deux fonctions. D'une part, sachant qu'en fonctionnement, une partie du liquide contenu dans la buse **4** part sous la forme de brouillard, il est nécessaire de réapprovisionner la buse **4** en liquide. D'autre part, un remplissage continu de la buse **4** associé à la recirculation du liquide permet de stabiliser les conditions de fonctionnement du système **40** même en présence de fortes accélérations du système, comme on peut les trouver dans un véhicule terrestre, maritime ou aérien par exemple.

A cette fin, ladite buse **4** est alimentée en liquide par au moins un réservoir de liquide **6**, dit « réservoir primaire » ou « réservoir de collecte ». Une pompe **10** dite « pompe de circulation » reliée d'une part audit réservoir primaire de liquide **6** et d'autre part à la buse **4** (par l'intermédiaire d'un conduit **8**) permet de faire circuler en permanence le liquide dans la buse **4** et de générer un jet de liquide **17** à la sortie de l'orifice de sortie **15** de la buse **4.** Ladite pompe de circulation **10** est avantageusement située directement au-dessus du réservoir de collecte **6**, comme cela est visible sur les figures, afin d'éviter autant que possible son désamorçage par manque d'eau.

L'admission du liquide en provenance du réservoir primaire de liquide **6** dans la buse **4** se fait à travers au moins un orifice d'admission **14.** De manière préférée, une pluralité d'orifices d'admission **14** sont aménagés autour de l'axe longitudinal de la buse **4** dans une zone proche de l'élément céramique piézo-électrique **1.**

Le système de nébulisation **40** selon l'invention comprend une chambre de mise en pression **5** qui communique avec la buse **4** par au moins un orifice **14** d'admission d'eau dans la buse. Selon l'invention, cette chambre de mise en pression **5** a un certain volume intérieur par rapport au volume de la buse **4**, ce qui assure une meilleure stabilité de la pression d'eau dans la buse **4** dans des conditions perturbées comme décrites ci-dessus ; cela sera expliqué ci-dessous de manière détaillée en relation avec la figure 5.

Selon l'invention, cette chambre de mise en pression **5** peut avoir différentes formes. Dans une variante montrée sur la figure 1, la buse **4** est réalisée avec une double paroi, la paroi interne **21** étant la paroi proprement dit de la buse **4**, apte à réfléchir les ondes acoustiques, comme décrit ci-dessus, et la paroi externe **22** renfermant avec la paroi **21** de la buse **4** un volume qui forme ladite chambre de mise en pression **5.** La chambre de mise en pression **5** enfermée entre sa paroi externe **22** et la paroi **21** de la buse **4** est reliée, d'une part, à l'intérieur de la buse **4** par au moins un orifice d'admission **14** de liquide (et de préférence, comme indiqué ci-dessus, par une pluralité d'orifices d'admission **14** aménagées de manière radiale, par exemple quatre orifices), et d'autre part au réservoir de collecte **6** par l'intermédiaire du conduit **8.**

Dans le mode de réalisation de l'invention illustré sur la figure 1, le réservoir de collecte **6** et la buse **4** forment un élément monobloc. Cela permet de simplifier sa construction ; un tel élément monobloc est plus robuste et résiste mieux à l'environnement perturbé d'un véhicule.

L'orifice de sortie **15** de la buse **4** a de préférence une forme circulaire. Dans une mode de réalisation, son diamètre est compris entre 3 et 8 mm, et avantageusement entre 4 et 6 mm ; la longueur intérieure de la buse est comprise entre 25 mm et 42 mm, sachant que cette distance correspond au champ proche des ultrasons générés par la céramique piézo-électrique **1.** A titre d'exemple, on peut utiliser une buse de hauteur 38 mm, avec un orifice de sortie d'un diamètre de 6 mm. La section d'admission de la buse **4** (i.e. la somme des surfaces des orifices d'admission **14**) doit être supérieure à la section de l'orifice de sortie **15** (de préférence au moins trois fois supérieure) afin d'éviter le phénomène de cavitation dans la buse **4** (ainsi qu'un manque d'eau). Cette condition est remplie par exemple avec quatre orifices d'admission **14** d'un diamètre de 5 mm pour un orifice de sortie **15** d'un diamètre de 6 mm.

En particulier pour l'utilisation du système de nébulisation **40** dans un véhicule, on prévoit avantageusement que le jet de liquide **17** généré à la sortie **15** de la buse **4** se vide dans un tube de collecte **7** dont l'axe longitudinal est de préférence incliné par rapport à la verticale. Le tube de collecte **7** peut être traversé par un flux d'air généré par un moyen de ventilation (non montré sur les figures), qui est de préférence réglable en débit et qui est situé en amont, en aval ou à l'intérieur du tube de collecte **7**. Ledit flux d'air entre dans le système de nébulisation **40** par une entrée d'air **11** et emporte les micro-gouttelettes d'eau **18** générées par la buse **4** autour du jet d'eau **17**. Ainsi se forme un brouillard **19** de micro-gouttelettes qui quitte le tube de collecte **7** par sa sortie **12** et entre dans son environnement de destination, par exemple l'habitacle d'un véhicule. Le jet d'eau **17** se projette contre la paroi interne du tube de collecte **7**, et le liquide ainsi recueilli se jette dans le bac à eau **6**. Ainsi le tube de collecte **7** sert également comme tube de guidage de la diffusion du brouillard. Ce mode de réalisation peut convenir également pour un système de nébulisation **40** stationnaire, notamment un système monté sur un étal.

Dans un mode de réalisation de l'invention, le dispositif comprend, en plus du réservoir primaire de collecte **6**, un réservoir de liquide dit secondaire (non représenté sur les figures), qui peut être déporté et relié au circuit de liquide représenté par le réservoir primaire de collecte **6** et la buse **4** par un conduit. Cela permet de diminuer la taille et l'encombrement du réservoir primaire **6**. Ledit réservoir de liquide secondaire peut être en tout matériau approprié, qui peut être souple, rigide ou semi-rigide. Il peut notamment être en métal (notamment aluminium, acier inoxydable) ou en plastique (notamment PE et PP). Comme il sera expliqué en plus grand détail ci-dessous, le réservoir secondaire peut comporter ou contenir une résistance chauffante ou plus généralement un moyen de chauffe, afin d'assurer l'hygiène, notamment bactériologique, de ce volume d'eau en chauffant l'eau et/ou les parois à une température suffisante pour détruire au moins partiellement des germes pathogènes, et plus généralement pour désinfecter et/ou sécher l'ensemble du dispositif **40.**

D'une manière générale, l'élément de céramique piézo-électrique **1** est de préférence de forme cylindrique, typiquement une plaquette de forme circulaire. A titre d'exemple, il peut avoir un diamètre de 20 mm ou de 25 mm. La fréquence d'ultrasons est avantageusement comprise entre 1,3 kHz et 2,3 kHz. Elle peut être par exemple de 1,68 MHz.

Dans un mode de réalisation, l'élément de céramique piézo-électrique **1** est fixé sur la paroi externe **22** de la chambre de mise en pression **5** à la base **23** de la buse **4** par un support **3** ; un joint **2** assure l'étanchéité entre ladite chambre de mise en pression **5** et le support **3**.

Ledit élément piézo-électrique **1** peut absorber une puissance électrique importante, par exemple 40 W pour un diamètre de 20 mm. Environ 40% de cette puissance est rendue sous forme d'énergie acoustique transmise au liquide, le reste est dissipé sous forme thermique. Pour cette raison, pendant son fonctionnement, l'élément piézo-électrique **1** doit être constamment refroidi par le liquide afin d'éviter sa détérioration par surchauffe. Les inventeurs se sont rendus compte que lorsque l'élément piézo-électrique **1** fonctionne à sec même pour une très courte durée, il risque d'être endommagé ou même être détruit. Pour éviter cela, les inventeurs ont prévu que le système de nébulisation **40** comporte des moyens appropriés permettant d'empêcher que ledit élément piézoélectrique **1** ne fonctionne (i.e. n'émette pas d'ondes acoustiques ou seulement des ondes acoustiques de très faible puissance) lorsque l'élément piézo-électrique **1** n'est pas immergé dans le liquide à pulvériser. Ces moyens peuvent prendre différentes formes, et comprennent en général au moins un moyen de détection du manque de liquide et/ou un moyen de détection de l'échauffement de l'élément piézo-électrique **1**, et un moyen de rétroaction sur l'alimentation électrique dudit élément piézo-électrique **1**.

Ledit moyen de détection du manque de liquide peut être un capteur de niveau **9** ou un capteur de présence qui coupe ou régule le fonctionnement de l'élément piézo-électrique **1**. Ce capteur **9** peut être un capteur optique ou un capteur capacitif ou encore un capteur inductif, mais parmi ces trois, on préfère un capteur optique qui présente une meilleure fiabilité. Ce capteur **9** peut se situer à différents endroits, notamment dans le récipient de collecte, ou à l'intérieur de la buse **4**, ou encore dans la chambre de mise en pression **5** de la buse **4**. Dans un mode de réalisation on utilise un capteur situé dans le réservoir primaire **6**. On peut également utiliser un capteur à ultrasons, agissant comme un capteur analogique permettant de faire la mesure du débit instantané du système.

Ledit moyen de détection du manque de liquide peut être un capteur qui détecte la présence du jet de liquide **17** en sortie de l'orifice de sortie **15** de la buse **4**. Ce moyen est moins préféré car il entraîne un retard dans la détection d'un défaut d'immersion dudit élément piézo-électrique **1**.

Ledit moyen de détection du manque de liquide peut être un capteur de pression dans la buse **4** et/ou dans la chambre de mise en pression **5** et/ou à la sortie de la pompe de circulation **10** et/ou dans le conduit **8**.

Un autre moyen pour détecter le manque de liquide dans la buse **4** est un détecteur de la température à la surface et/ou à l'intérieur dudit élément piézo-électrique **1**, ce qui permet de détecter l'échauffement rapide dudit élément piézo-électrique **1** avant qu'il n'ait pris dommage. Cette détection peut se faire par exemple à l'aide d'un thermocouple.

Dans le cadre de la présente invention la détection thermique au niveau de l'élément piézo-électrique **1** n'est cependant pas un mode de réalisation préféré : on préfère les moyens qui détectent de manière plus directe le manque de liquide, et à un stade plus précoce auquel le manque de liquide n'a pas encore perturbé le fonctionnement dudit élément piézo-électrique **1.**

On peut combiner deux ou plusieurs moyens de détection, sélectionnés parmi ceux qui viennent d'être présentés et/ou parmi ceux qui le seront ci-dessous.

Différents types de pompes peuvent être utilisés pour la pompe de circulation **10.** Elle doit être à débit réglable ; une pompe réglable entre 0,1 et 2,8 litres/min convient pour une buse **4** qui présente les dimensions indiquées ci-dessus. Dans un mode de réalisation avantageux, qui convient bien à un système miniaturisé utilisable pour l'habitacle d'un véhicule, la pompe de circulation **10** peut être une pompe hélice. Avantageusement, cette pompe absorbe un courant continu et on règle la tension pour faire varier la vitesse de rotation et donc la pression de refoulement en sortie de la buse **4,** ce qui permet de modifier la longueur du jet **17.**

D'une manière générale, un système tel que décrit ci-dessus présente le risque que la pompe de circulation **10** aspire temporairement de l'air plutôt que du liquide lorsque le niveau d'eau dans le réservoir de collecte **6** change fortement, par exemple suite à l'accélération, au freinage ou au basculement brusque du véhicule. Il y a également un risque de formation de bulles d'air dans le réservoir de collecte **6** si l'environnement est très perturbé ; ces bulles d'air peuvent être avalées par la pompe **10.** L'aspiration d'air peut même conduire au désamorçage temporaire ou permanent de la pompe. Cela risque de conduire à une baisse de la pression d'eau dans la buse **4** et à un manque de liquide tel que l'élément piézo-électrique **1** n'est plus immergé.

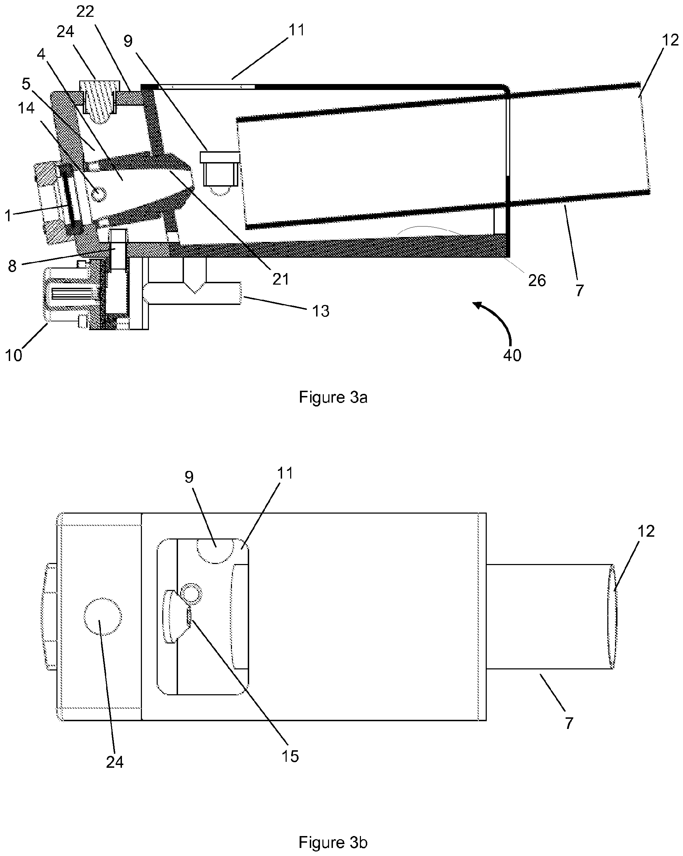

Pour éviter un manque de liquide dans la buse **4,** on propose trois moyens qui peuvent être combinés (et auxquels peuvent s'ajouter le cas échéant les moyens de détection de manque de liquide présentés ci-dessus) : Le réservoir de collecte **6** peut avoir une forme caractérisée par un fond **26** au moins partiellement incliné dont le point bas est proche de son orifice d'évacuation **25** ; cette forme peut être une forme d'entonnoir comme sur la figure 1 ou une autre forme comme sur les figures 3a et 5. Par ailleurs, comme illustré sur les figures 1, 2 et 5, on peut compartimenter le réservoir de collecte **6** par au moins une plaque **16** dite « plaque de stabilisation » qui a pour effet de stabiliser le niveau de liquide, et de préférence d'une pluralité de telles plaques de stabilisation, qui peuvent être disposées dans le sens vertical, horizontal ou incliné. Ladite plaque de stabilisation **16** est une plaque comportant au moins une ouverture **20** à travers laquelle peut s'écouler le liquide. Le troisième moyen annoncé ci-dessus est lié au volume de la chambre de mise en pression **5** ; il sera présenté ci-dessous en relation avec la figure 5.

Comme indiqué ci-dessus, le réservoir de collecte **6** peut comporter au moins une plaque de stabilisation **16** du niveau de liquide qui est sensiblement horizontale qui s'étale sur toute ou partie de la largeur du réservoir, et il peut, également ou en plus, comporter au moins une plaque qui n'est pas horizontale, par exemple une plaque verticale, qui s'étale sur toute ou partie de la hauteur dudit réservoir. Le réservoir peut comporter des plaques qui sont sensiblement parallèles. De préférence, les ouvertures **20** de deux plaques **16** parallèles voisines ne se trouvent pas au même endroit, c'est-à-dire ne se superposent pas, mais sont décalés dans le plan de la plaque.

La figure 1 montre un exemple d'un réservoir de collecte **6** en forme d'entonnoir pourvu de trois plaques **16** de stabilisation du niveau de liquide horizontales et parallèles ; leurs ouvertures **20** ne se superposent pas. Le réservoir de collecte **6** peut comporter une évacuation du liquide (non montrée sur les figures) dans sa partie basse qui constitue avantageusement le point le plus bas du système de nébulisation ; ainsi on minimise le volume mort en cas d'arrêt du système **40,** après son vidage par ce point le plus bas.

Pour améliorer encore l'effet des plaques de stabilisation **16,** au moins une partie desdites ouvertures **20** peuvent être obturées au moins en partie par un clapet qui s'ouvre au moins partiellement sous une pression d'eau venant d'un côté et se ferme sous une pression d'eau venant du côté opposé, ou s'ouvre moins largement sous une pression d'eau venant d'un côté que sous une pression d'eau équivalente venant du côté opposé.

Dans une variante qui peut être combiné avec les clapets, lesdites plaques de stabilisation **16** peuvent être réalisées toutes en en partie sous la forme de grilles ou de passoires.

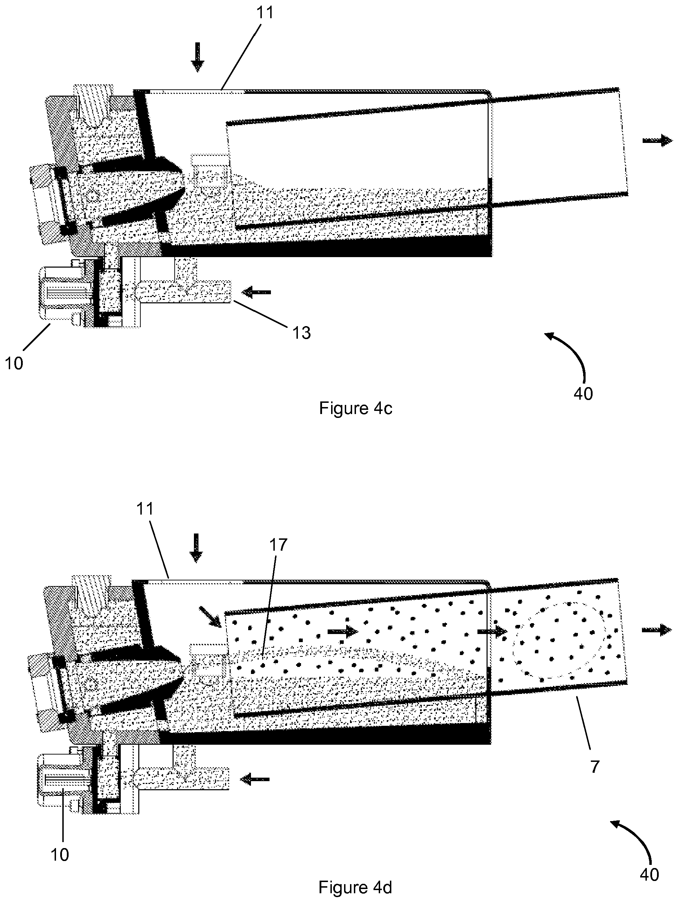

On peut également positionner au moins une plaque de stabilisation **16** dans le tube de guidage **7** comme cela est montré schématiquement sur la figure 4d. Cela résout un problème qui est propre aux systèmes de nébulisation dont la buse **4** présente un axe longitudinal incliné par rapport à la verticale et dont le jet de liquide **17** est collecté dans un tube de guidage **7** : il y a un risque de formation de bulles d'air lors de la collecte du liquide du jet ; ces bulles peuvent se retrouver dans le réservoir de collecte **6** et être aspirées par la pompe de circulation **10,** dont elles sont susceptibles de perturber le bon fonctionnement. Les inventeurs ont trouvé qu'une plaque de stabilisation **16** disposée dans le tube de guidage **7** peut diminuer la formation et le transport de bulles d'air.

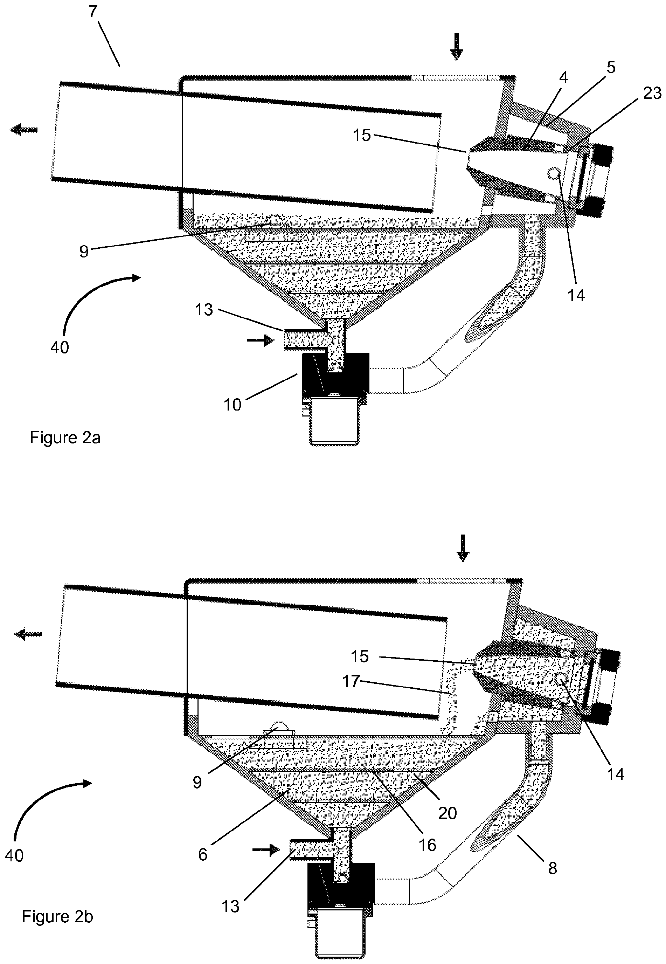

Les figures 2a à 2d montrent de manière schématique et successive la mise en route et le fonctionnement du dispositif **40** selon l'invention présenté sur la figure 1. Comme montré sur la figure 2a, le système **40** est rempli en faisant entrer du liquide par l'orifice d'entrée **13.** Le remplissage peut être réalisé à travers une électrovanne ou par une pompe péristaltique ou d'autres types de pompe (piston, membrane ....) (non montrées sur la figure) situées en amont de l'entrée **13** ; ce remplissage fait monter le niveau de liquide dans le réservoir **6** et le conduit **8** jusqu'à un point détecté par un capteur de présence d'eau **9** à partir duquel la pompe de circulation **10** est mise en route.

Comme montré sur la figure 2b, le remplissage progressif du réservoir **6** par l'orifice d'entrée **13** et la pression générée par la pompe de circulation **10** font que le liquide envahit la chambre de mise en pression **5** puis la buse **4,** et un jet d'eau court **17** sort de l'orifice de sortie **15.**

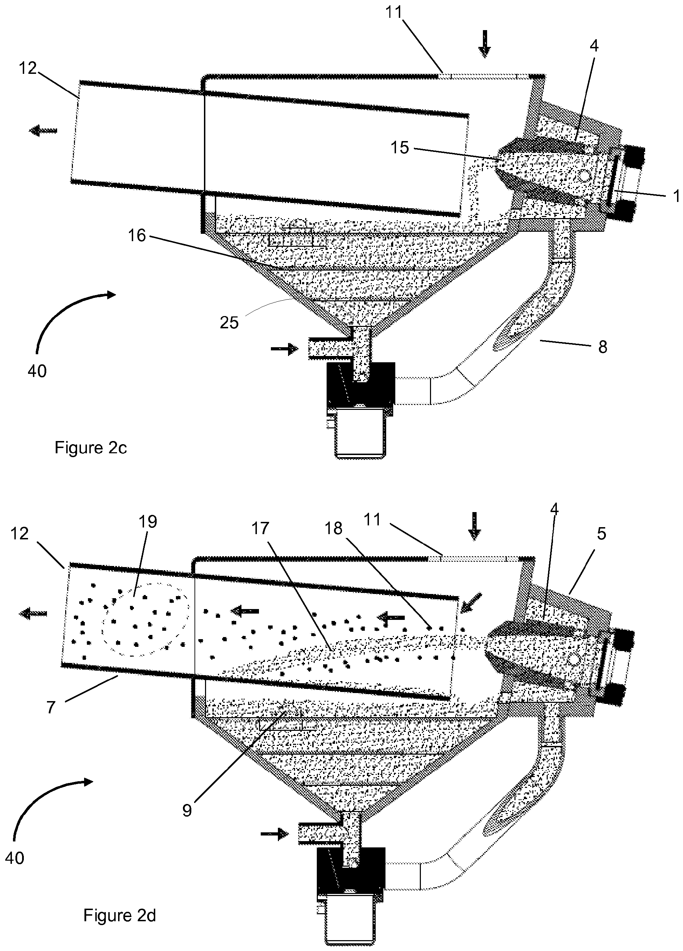

Lorsque le niveau de liquide dans le réservoir **6** est de nouveau suffisant (tel que détecté par exemple par le capteur de présence d'eau **9**) et l'élément piézo-électrique complètement immergé, voir la figure 2c, l'alimentation électrique de l'élément piézo-électrique **1** est activée. La céramique **1** est excitée à sa fréquence de résonance, ce qui a pour conséquence de générer une onde acoustique qui est canalisée par la buse **4** agissant, grâce à la forme spécifique de sa paroi interne, comme concentrateur d'onde acoustiques. Comme montré sur la figure 2d, le jet d'eau **17** se rallonge sous l'effet des ondes acoustiques jusqu'à s'écouler dans le tube de guidage **7**, et des micro-gouttelettes **18** d'eau sont arrachées par l'onde acoustique. Sous l'effet du flux d'air (représenté par les flèches), un brouillard **19** se forme ; il quitte le tube de guidage **7** par sa sortie de nébulisation **12**. Le jet d'eau **17** est recueilli par le tube de guidage **7** et l'eau est collectée dans le récipient de collecte **6** pour être recyclée dans le système **40**.

Lorsque le niveau d'eau tel que détecté par le capteur de présence d'eau **9** est insuffisant pour assurer que l'élément piézo-électrique **1** est totalement immergé, une boucle de rétroaction interrompt ou diminue le fonctionnement de l'élément piézo-électrique **1**. Si cette baisse de niveau se prolonge au-delà d'une certaine durée, de l'eau est rajoutée par l'entrée de remplissage **13**, si possible, par exemple à partir dudit réservoir secondaire. L'ajout d'eau peut aussi se faire de manière permanente, de manière continue ou discontinue, par exemple à l'aide d'une pompe péristaltique (non montrée sur les figures), afin de compenser la perte d'eau due à la nébulisation.

La figure 3 montre un autre mode de réalisation de l'invention qui se distingue de celui de la figure 1 par la forme du réservoir primaire de collecte **6** et par la forme de la chambre de mise en pression **5** (qui sera expliquée en plus grand détail en relation avec la figure 5). Le dispositif de nébulisation **40** selon la figure 3 est équipé d'un deuxième capteur **24** de la présence d'eau qui se situe dans la chambre de mise en pression **5**, de préférence dans la partie supérieure de celle-ci ; ce deuxième capteur **24** est optionnel.

Plus précisément, le réservoir primaire de collecte présente une forme caractérisée par un fond au moins partiellement incliné dont le point bas est proche de son orifice d'évacuation **25**, mais cette forme n'est pas celle d'un entonnoir comme dans la figure 1. Le dispositif selon la figure 3 ne montre pas de plaques de stabilisation **16**, mais celles-ci peuvent être ajoutées (par exemple d'une manière analogue à ce qui est montré sur la figure 5b).

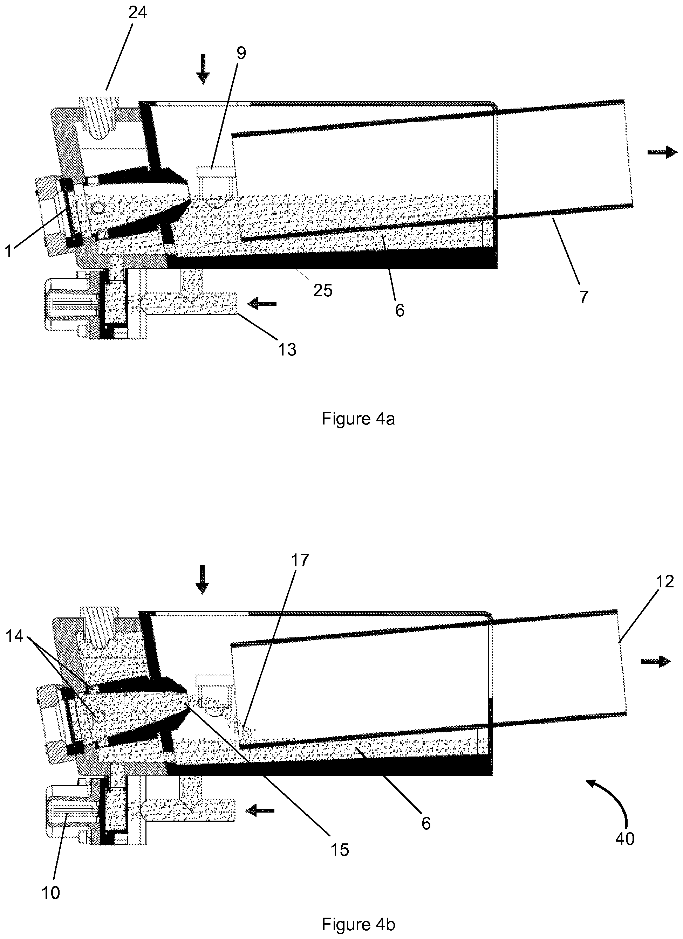

Les figures 4a à 4d montrent de manière schématique et successive la mise en route et le fonctionnement du dispositif **40** selon l'invention présenté sur la figure 3 ; ce procédé est similaire à celui expliqué ci-dessus en relation avec les figures 2a à 2d. Comme montré sur la figure 4a, le système **40** est rempli en faisant entrer du liquide par l'orifice d'entrée **13**. Comme dans le cas de la figure 2a, le remplissage peut être réalisé à travers une électrovanne ou par une pompe péristaltique ou d'autres types de pompe (piston, membrane....) (non montrées sur la figure) situées en amont de l'entrée **13** ; ce remplissage fait monter le niveau de liquide dans le réservoir **6** et le conduit **8** jusqu'à un point détecté par un capteur de présence d'eau **9** à partir duquel la pompe de circulation **10** est mise en route.

Comme montré sur la figure 4b, le remplissage progressif du réservoir **6** par l'orifice d'entrée **13** et la pression générée par la pompe de circulation **10** font que le liquide envahit la chambre de mise en pression **5** puis la buse **4**, et un jet d'eau court **17** sort de l'orifice de sortie **15**.

Lorsque le niveau de liquide dans le réservoir **6** est de nouveau suffisant (tel que détecté par exemple par le capteur de présence d'eau **9**) et l'élément piézo-électrique complètement immergé, voir la figure 4c, l'alimentation électrique de l'élément piézo-électrique **1** est activée. La céramique **1** est excitée à sa fréquence de résonance, ce qui a pour conséquence de générer une onde acoustique qui est canalisée par la buse **4** agissant, grâce à la forme spécifique de sa paroi interne, comme concentrateur d'onde acoustiques. Comme montré sur la figure 4d, le jet d'eau **17** se rallonge sous l'effet des ondes acoustiques jusqu'à s'écouler dans le tube de guidage **7**, et des micro-gouttelettes **18** d'eau sont arrachées par l'onde acoustique. Sous l'effet du flux d'air (représenté par les flèches), un brouillard **19** se forme ; il quitte le tube de guidage **7** par sa sortie de nébulisation **12**. Le jet d'eau **17** est recueilli par le tube de guidage **7** et l'eau est collecté dans le récipient de collecte **6** pour être recyclée dans le système **40**.

Lorsque le niveau d'eau tel que détecté par le capteur de présence d'eau **9** est insuffisant pour assurer que l'élément piézo-électrique **1** est totalement immergé, une boucle de rétroaction interrompt ou diminue le fonctionnement de l'élément piézo-électrique **1**. Si cette baisse de niveau se prolonge au-delà d'une certaine durée, de l'eau est rajoutée par l'entrée de remplissage **13**, si possible, par exemple à partir dudit réservoir secondaire. L'ajout d'eau peut aussi se faire de manière permanente, de manière continue ou discontinue, par exemple à l'aide d'une pompe péristaltique (non montrée sur les figures), afin de compenser la perte d'eau due à la nébulisation.

Dans tous les modes de réalisation, on peut prévoir un deuxième capteur de présence d'eau **24** en haut de la chambre de mise en pression **5** : si ce capteur indique un niveau insuffisant de liquide, une boucle de rétroaction peut couper ou diminuer l'alimentation de l'élément piézo-électrique **1** et/ou augmenter le débit de la pompe de circulation **10**. Ainsi, le volume de la chambre permet d'assurer une protection de l'élément piézo-électrique **1**, le temps que son alimentation soit coupée et que l'élément piézo-électrique **1** cesse de résonner.

La figure 5 montre un autre mode de réalisation de l'invention. Le tube de guidage **7** forme ici au moins en partie le réservoir de collecte **6**, de manière à ce que son fond incliné forme au moins en partie le fond incliné dudit réservoir de collecte **6.** Il comporte au moins une plaque de stabilisation **16**, et de préférence (comme sur la figure 5) une pluralité de plaques de stabilisation **16**, disposées par exemple horizontalement et/ou verticalement. Ce mode de réalisation permet une construction particulièrement compacte du système de nébulisation **40** et dont la tolérance aux perturbations mécaniques et excellente. Il n'est pas gênant si en cas de très grande perturbation mécanique (choc) le niveau de liquide du réservoir de collecte **6** déborde occasionnellement et temporairement au-dessus du niveau de l'orifice de sortie **14** de la buse **4**, de manière à noyer le jet d'eau **17** : cela interrompt momentanément la production de brouillard **19**, mais ne met pas en péril l'élément céramique, et ne conduira pas à un effet perceptible pour l'utilisateur du système.

La figure 5 illustre un aspect essentiel de l'invention qui est expliqué ici en détail. Il est lié aux relations entre différents volumes. On désigne par V1 le volume de liquide dans le réservoir de collecte **6**, par V2 le volume de liquide dans la pompe de circulation **10**, par V3 le volume de liquide dans la chambre de mise en pression **5** en partie inférieure de la buse **4** (i.e. inférieur à la hauteur qui définit le plan inférieur du volume V5, voir ci-dessous) et par V4 le volume de liquide dans la buse **4**. Le volume V3 peut être très faible voire nul.

On désigne par V5 le volume de liquide dans la partie supérieure de la chambre de mise en pression **5** se situant à un niveau de liquide supérieur au plus haut des trois points suivants : l'orifice **14** d'admission d'eau de la buse situé le plus haut, ou le bord supérieur de l'orifice **15** de sortie de la buse **4**, le point le plus haut de la céramique **1** piézo-électrique. Ainsi quelle que soit l'inclinaison $\alpha$ de la buse **4**, tout point de la céramique piézo-électrique **1** se trouve à un niveau inférieur au volume V5.

En fonctionnement normal du système **40** (voir par exemple les figures 2d et 4d), les volumes V1, V2, V3, V4 et V5 sont remplis de liquide, la pompe de circulation **10** et l'élément piézo-électrique **1** fonctionnent et génèrent un jet d'eau **17** de longueur approximativement constante, ce qui illustre l'état stationnaire du système.

Selon l'invention, on dimensionne la chambre de mise en pression **5** de manière à ce qu'elle présente un volume tampon (volume de sécurité) V5 suffisant par rapport au volume V4 de la buse **4**, de manière à ce que dans le cas où la pompe de circulation **10** ne pompe plus de liquide (par exemple lorsque le niveau de liquide dans le réservoir de collecte **6** est insuffisant, ou lorsque la pompe de circulation **10** est désamorcée), le volume V5 assure durant un certain laps de temps $t_s$ l'alimentation en eau du volume V4 de la buse **4**, de manière à ce que l'élément piézo-électrique **1** soit encore noyé pendant ce laps de temps $t_s$. Ce laps de temps $t_s$ peut être, tout ou en partie, mis à profit pour couper l'alimentation de l'élément piézo-électrique **1**, et/ou pour attendre si le niveau de liquide se rétablit tout seul (notamment en cas de perturbation mécanique ou lorsque la pompe de circulation **10** a simplement avalé une bulle d'air). Le temps $t_s$ doit être suffisamment long pour permettre la coupure totale de l'alimentation de l'élément piézo-électrique **1** et l'arrêt de son fonctionnement ; la demanderesse a en effet observé que l'arrêt du fonctionnement de l'élément piézo-électrique **1** n'est pas instantané lorsque l'on coupe son alimentation électrique : l'élément piézo-électrique **1** continue à vibrer pendant que les circuits de son alimentation électrique se vident.

D'une manière générale, on préfère dans le cadre de la présente invention que le rapport des volumes V5/V4 soit d'au moins 2 et de préférence d'au moins 6, et encore plus préférentiellement d'au moins 12.

D'une manière plus précise, on prend en considération le temps de réaction souhaitable du système pour couper l'alimentation électrique de l'élément piézo-électrique **1** en cas de manque d'eau. Il n'est pas forcément souhaitable de couper l'alimentation à la moindre baisse de niveau dans la chambre de mise en pression **5**, ce qui risque de conduire à une génération de brouillard trop intermittente. Mais il faut être sûr que lorsque cette baisse se prolonge ou s'aggrave au-delà d'une certaine durée, l'alimentation électrique de l'élément piézo-électrique **1** soit coupée ou au moins fortement réduite. Ainsi, les inventeurs considèrent que dans un environnement mécaniquement instable (véhicule, étal entouré par une foule de personnes) le système de nébulisation **40** selon l'invention doit permettre un fonctionnement de l'élément piézo-électrique **1** pendant une durée $t_s$ comprise entre **1** et 10 secondes sans alimentation en liquide par la pompe de circulation **10**, et de préférence entre 2 et 5 secondes.

Dans ce contexte, un paramètre important est le débit de liquide généré par l'élément piézo-électrique **1** à la sortie de l'orifice **14** de la buse **4** en l'absence de pompage par la pompe de circulation **10** ; ce débit (qui se manifeste souvent par la présence d'un petit jet d'eau appelé « fontaine acoustique ») dépend (pour un angle $\alpha$ de positionnement de la buse **4** et un liquide donnés) essentiellement de la puissance de l'élément piézoélectrique **1**.

De manière encore plus précise, le débit de la fontaine acoustique peut être exprimé par

$$Q_{piezo} = K \times P_{max}$$

où $P_{max}$ est la puissance électrique maximale consommée par l'élément piézoélectrique et $Q_{piezo}$ est le débit de la fontaine acoustique à cette puissance $Q_{piezo}$, et K est un facteur de proportionnalité. On souhaite une durée de fonctionnement de sécurité de $t_s$ secondes, c'est-à-dire que lorsque la pompe de circulation **10** cesse de fonctionner (notamment par désamorçage), le système dispose d'un délai d'environ $t_s$ secondes pour couper l'alimentation de l'élément piézoélectrique **1.** Avantageusement, le délai $t_s$ est compris entre 1 et 10 secondes, et on préfère une valeur entre 2 et 5 secondes. Selon l'invention, cet objectif peut être atteint en prévoyant un volume tampon de sécurité V5 suffisant, qui correspond au volume de la chambre de mise en pression **5** se situant à un niveau de liquide supérieur au bord supérieur de l'orifice **15** de sortie de la buse **4.** Ce volume doit être supérieur au volume V4 de la buse **4.**

On souhaite donc que

$$V5 \geq V4 + Q_{piezo} \times t_s.$$

Cette relation peut être exprimée par

$$V5 \geq V4 + K \times P_{max} \times t_s.$$

Dans un exemple typique on utilise une buse **4** avec un volume V4 de 0,0054 litres, et $Q_{piezo}$ est 50 W pour une tension d'alimentation de 22 V avec un rendement acoustique d'environ 40% ; l'angle est $\alpha$ compris entre 0 et 30°. Dans ces conditions $P_{max}$ est d'environ 1,5 litres/min, et par conséquent K = 0,0005 l/Ws. Si l'on vise une valeur $t_s$ = 5 secondes, V5 doit être au moins égal à 0,13 litres. Le rapport V5/V4 est donc de 24. Comme indiqué ci-dessus, la valeur $t_s$ peut être inférieure à 5 secondes, ce qui tend à diminuer le rapport V5/V4.

On peut estimer le rapport V5/V4 encore d'une manière plus précise, qui prend en compte notamment la valeur de l'angle $\alpha$ et les sections des orifices **14,15.** On constate cependant que sauf situation extrême (notamment : angle $\alpha$ inférieur à -30°, rapport des sections de l'orifice de sortie **15** et des orifices d'admission **14** trop faible), l'ordre de grandeur du résultat ne change pas. Afin de ne pas alourdir la présente description nous présentons en plus grand détail une estimation plus précise dans l'annexe ci-dessous.

Un autre problème d'un système de nébulisation **40** est son alimentation en liquide. Dans le cas où il est installé dans un véhicule, il est exclu de faire assurer l'alimentation en eau du système par un opérateur humain (utilisateur du véhicule, technicien etc.), à l'instar du système de lave-glace d'un véhicule. Dans le cas où le système de nébulisation **40** est installé sur un étal de vente, une alimentation en eau externe n'est pas toujours disponible, ou ne peut être monopolisée par un branchement permanent au système de nébulisation **40 ;** on peut prévoir dans ce cas un réservoir secondaire de liquide de capacité suffisante qui peut être rempli par un opérateur (par exemple tous les matins ou une fois par semaine). De manière avantageuse, le système de nébulisation **40** selon l'invention est alimenté en eau par un système de récupération d'eau de provenance extérieure audit système de nébulisation.

Cette eau de récupération peut entrer dans le système de nébulisation **40** par ledit réservoir secondaire de liquide. Il peut s'agir par exemple de l'eau de condensation qui se forme sur des surfaces des matériaux en contact avec l'eau de fonte de glace utilisée pour la réfrigération directe des produits frais (par exemple poisson, fruits de mer) exposés sur l'étal. Elle peut également provenir d'un système de climatisation, et plus spécialement de l'eau de condensation issue dudit système de climatisation. Elle peut être admise de manière continue ou discontinue dans le système de nébulisation **40.** Quelle que soit l'origine de l'eau de récupération, elle doit être purifiée avant d'entrer dans la buse **4.** La purification peut être réalisée par chauffage à une température suffisante pour une durée suffisante (ce chauffage peut être réalisé de manière permanente ou intermittente, par exemple par une résistance chauffante), et/ou par un élément filtrant (par exemple un filtre céramique capable d'éliminer des particules de taille supérieure à 1 $\mu$m et de préférence supérieure à 0,5 $\mu$m). Pour alimenter l'élément filtrant en eau à purifier, une pompe (par exemple une pompe péristaltique) peut être prévue qui génère une pression suffisante pour vaincre la perte de charge causée par l'élément filtrant.

Encore un autre problème est le vidage du système de nébulisation **40** : on ne souhaite pas qu'en cas de non fonctionnement prolongé, l'eau stagne dans des parties du système, car cela est susceptible de favoriser la prolifération de germes pathogènes. Pour cela, différentes variantes sont proposées ici.

Dans un mode de réalisation avantageux, le vidange du liquide se fait par un orifice aménagé dans la partie basse du réservoir de collecte **6.** Cet orifice peut être le même que celui par lequel arrive l'eau pour remplir ledit réservoir de collecte **6** (dans cette variante il s'agit donc de l'orifice portant le repère **13**). Une électrovanne peut être prévue, et/ou une pompe d'aspiration et/ou de remplissage.

Dans une variante on remplit le réservoir de liquide primaire **6** par une pompe réversible à partir du réservoir de liquide secondaire. Dans ce cas, il est possible de vider le réservoir primaire **6** dans le réservoir secondaire par cette même pompe ; le réservoir secondaire peut ensuite être vidé dans le système de climatisation avec lequel il communique par un conduit.

Dans un autre mode de réalisation, qui convient en particulier à l'utilisation du système de nébulisation **40** sur un étal dans un lieu de vente, le système dispose d'une alimentation externe en eau sous pression. Compte tenu du fait qu'il est d'une manière générale plus facile d'installer une arrivé d'eau sous pression qu'une évacuation d'eau, il serait souhaitable de ne pas avoir impérativement besoin d'une évacuation d'eau pour un tel système de nébulisation. Selon l'invention, le système de nébulisation **40** comprend des moyens de chauffage permettant son vidage par évaporation complète du liquide. La vapeur d'eau générée ainsi peut être aussi utilisée pour la décontamination au moins partielle du système (notamment des gaines et tuyaux de circulation du brouillard et du tube de guidage de la diffusion **7**, du volume V1 ainsi que des volumes V2, V3, V4 et V5).

De manière préférée, ce moyen de chauffage est une résistance chauffante qui ne craint pas la surchauffe si elle est utilisée à sec ; une résistance chauffante enrobée de silicone de type connu peut convenir. Cette résistance chauffante peut être installée à l'intérieur du réservoir de collecte **6.** Elle peut être utilisée de différentes manières. Notamment, elle peut chauffer de manière intermittente l'eau à une température suffisante pour tuer certains types de germes pathogènes, tels que les germes provoquant la légionellose contenus dans le liquide du système et sur ses parois. Elle peut aussi être utilisée pour évaporer l'ensemble du liquide stagnant dans le système lors d'un arrêt prolongé. Cela évite l'utilisation de produits bactéricides. A cette fin, la température doit pouvoir atteindre au moins 70°C, comme décrit dans la demande de brevet WO 2011/039487 de la demanderesse.

Le dispositif **40** selon l'invention, grâce à sa buse **4** à focalisation d'ondes acoustiques générées par un élément piézo-électrique **1**, est apte à créer et répandre un brouillard formé de gouttelettes d'un diamètre moyen typique compris entre 0,5 $\mu$m et 10 $\mu$m, de préférence entre 1 $\mu$m et 5 $\mu$m. Cette taille de particules dépend notamment de la fréquence des ondes acoustiques produites par ledit élément piézo-électrique **1.** Il peut être utilisé à bord de tout type de véhicule, notamment terrestre, aérien ou maritime. Il peut y être utilisé notamment pour rafraîchir et/ou humidifier et/ou parfumer et/ou désinfecter l'air d'un habitacle (par exemple d'une cabine de wagon ferroviaire, de navire ou d'un aéronef) ou d'un volume de transport de produits. Il peut aussi être utilisé dans des applications stationnaires, par exemple dans un local commercial, artisanal, industriel ou dans un local d'habitation, pour rafraîchir et/ou humidifier et/ou désinfecter et/ou parfumer l'air ambiant. Il peut être utilisé notamment pour rafraîchir et/ou humidifier des produits (notamment des produits frais) exposés à la vente sur un étal. Ce dispositif **40** présente les avantages d'être particulièrement compact (grâce à sa conception avec buse **4** inclinée qui vide son jet d'eau dans le tube de guidage **7** de la diffusion du brouillard), particulièrement fiable (grâce à sa résistance aux perturbations mécaniques et grâce à sa conception qui résiste à une interruption du fonctionnement de la pompe de circulation 10 de quelques secondes) et de nécessiter peu de maintenance (grâce au système de récupération d'eau).

Annexe à la description :

Cette annexe montre en plus grand détail le calcul du rapport V5/V4 pour un dispositif selon l'invention qui a été réalisé par les inventeurs ; il sert également comme exemple. Dans cette annexe le paramètre $t_s$ est désigné par $t_{securité}$.et l'angle $\alpha$ est désigné par $\theta$.

Les paramètres physiques connus et mesurables sont les suivants :

- $S_s$ Section de sortie **15** de la buse **4** de concentration
- $S_e$ Section d'entrée **14** de la buse **4** de concentration
- H Hauteur de la buse **4** de concentration, de la base jusqu'à la section $S_s$
- $P_{électrique}$ Puissance électrique

Afin de limiter le phénomène de cavitation ainsi qu'un manque d'eau dans la buse 4, on fixe les conditions suivantes :

$$S_e \gg S_s \qquad , \qquad \text{idéalement il faut que} \qquad S_e \geq 3{,}5\, S_s$$

On sait que la vitesse de sortie du jet $v_{jet}$ **17** est liée à la puissance acoustique de l'élément piézo-électrique **1** ainsi qu'aux paramètres géométriques de la buse **4** :

$$v_{jet} = f(P_{acoustique} ; H ; S_s ; \theta)$$

Si on fixe les paramètres géométriques H, $S_s$, $\theta$ on obtient

$$v_{jet} = f(P_{acoustique} ; K_1)$$

avec indice $K_1$ constante.

De plus on sait que $P_{acoustique} = f(P_{électrique} ; K_2)$ avec indice $K_2$ constante.

On obtient $v_{jet} = f(P_{électrique} ; K_1 ; K_2)$ soit

$$v_{jet} = f(P_{électrique} ; K_3)$$

avec indice $K_3$ constante.

On donne ici un descriptif du fonctionnement en cas de désamorçage de la pompe de circulation **10**, notamment pour évaluer le rapport des volumes d'eau V5/V4 :

Les conditions initiales sont les suivantes (régime stationnaire) :

- Système rempli en eau.
- Pompe de circulation **10** et acoustique (génération du brouillard, excitation de la céramique **1**) en fonctionnement.
- Les volumes $V_1$, $V_2$, $V_3$, $V_4$ et $V_5$ sont quasiment constants.

En cas de désamorçage de la pompe **10** dû à une perturbation d'aspiration de l'eau dans le réservoir de collecte **6**, c'est-à-dire lorsque que la pompe de circulation **10** n'arrive plus à aspirer le volume $V_1$ et $V_2$ en eau, il faut que le volume $V_5$ (volume d'eau supérieur dans la chambre de mise en pression **5**) assure durant un temps $t_{sécurité}$ l'alimentation en eau du volume $V_4$ de la buse **4**.

On en déduit donc la relation suivante :

$$V_5 \geq Q_{jet} \cdot t_{sécurité}$$

$$V_5 \geq v_{jet} \cdot S_s \cdot t_{sécurité}$$

$$V_5 \geq K_3 \cdot P_{électrique} \cdot S_s \cdot t_{sécurité} \qquad \text{comme } S_e \geq 3 S_s$$

On peut aussi dire que :

$$V_5 \geq [K_3 \cdot P_{électrique} \cdot S_e \cdot t_{sécurité}] / 3$$

Un dispositif réalisé par les inventeurs est caractérisé par les paramètres suivants :

$$S_s = \frac{\pi \cdot d^2}{4} = \frac{\pi \cdot 6^2}{4} \text{ mm}^2$$

avec d = 6 mm pour le diamètre de l'orifice 15 de sortie de la buse 4.

On vérifie que

$$S_e \gg S_s, \qquad 4 \cdot \frac{\pi \cdot 5^2}{4} \gg \frac{\pi \cdot 6^2}{4} \text{ et } 100 \gg 36.$$

Nous savons que $K_3 = 0{,}02$ pour un diamètre de buse compris entre 4 et 8 mm et $\theta = 10°$ avec H = 38 mm (H désigne la hauteur intérieure de la buse **4**).

De plus, dans l'exemple $P_{électrique} = 50$ W (constant).

Si on fixe comme objectif $t_{sécurité} = 3$ sec, on obtient :

$$V_5 \geq K_3 \cdot P_{électrique} \cdot S_s \cdot t_{sécurité} \qquad \text{et} \qquad V_5 \geq 85 \text{ ml.}$$

Il faut donc que le volume $V_5$ soit au minimum de 85 ml pour assurer une alimentation en eau dans le volume de la buse $V_4$ durant 3 secondes.

**Revendications**

1. Dispositif de nébulisation (40) comportant

   a) une buse (4) de nébulisation pourvue d'au moins un orifice (14) d'admission de liquide et d'au moins un orifice de sortie de liquide (15), et au côté opposé dudit orifice de sortie (15) un élément piézo-électrique (1) apte à émettre des ondes acoustiques dans ledit liquide,
   et la section transversale de ladite buse (4) présentant un rétrécissement progressif en direction dudit premier orifice de sortie (15), de manière à ce que dans ladite buse (4) les ondes acoustiques soient focalisées pour créer un brouillard (19) de gouttelettes (18) dudit liquide ;
   b) un réservoir de collecte (6) qui alimente ladite buse (4) en liquide ;
   c) une pompe (10) dite « pompe de circulation » reliée d'une part au réservoir de collecte (6) et d'autre part à ladite buse (4) par le au moins un orifice (14) d'admission aménagé dans ladite buse (4), ladite pompe de circulation (10) étant apte à générer dans ladite buse (4) une pression de liquide suffisante pour maintenir un jet de liquide (17) sortant par ledit orifice de sortie (15) de la buse (4) ;
   d) une chambre de mise en pression (5) qui est traversée par le liquide sortant de la pompe de circulation (5) avant son entrée dans ladite buse (4),

   ledit dispositif de nébulisation étant **caractérisé en ce que** le volume (V5) de la partie supérieure de la chambre de mise en pression (5) se situant à un niveau de liquide supérieur au plus haut des trois points suivants : l'orifice (14) d'admission d'eau de la buse situé le plus haut, le bord supérieur de l'orifice (15) de sortie de la buse (4), le point le plus haut de l'élément piézo-électrique (1), est au moins deux fois (de préférence au moins six fois et encore plus préférentiellement au moins douze fois) plus grand que le volume (V4) de la buse (4).

2. Dispositif selon la revendication 1, **caractérisé en ce que** la section transversale de ladite buse (4) présente un rétrécissement tel que les ondes acoustiques soient focalisées au niveau dudit orifice de sortie (15).

3. Dispositif selon la revendication 1 ou 2, **caractérisé en ce que** la somme des surfaces des orifices d'admission (14) est supérieure, et de préférence au moins trois fois supérieure, à la section de l'orifice de sortie (15).

4. Dispositif selon l'une quelconque des revendications 1 à 3, **caractérisé en ce que** l'axe longitudinal de ladite buse (4) forme un angle d'inclinaison $\alpha$ par rapport à l'horizontale qui se situe entre 0° et 45°, de préférence entre 0° et 30° et encore plus préférentiellement entre 5° et 20°.

5. Dispositif selon l'une quelconque des revendications 1 à 4, **caractérisé en ce qu'**il comprend au moins une plaque de stabilisation (16) du niveau de liquide, disposée horizontalement, verticalement ou en biais, comportant chacune au moins une ouverture (20) et/ou formant au moins une ouverture (20) avec au moins une autre plaque de stabilisation (16) ou une paroi dudit réservoir (6).

6. Dispositif selon la revendication 5, **caractérisé en ce que** lesdites plaques stabilisation (16) sont au nombre d'au moins deux et sont disposées de manière à ce que les ouvertures (20) sont décalées les unes par rapport aux autres.

7. Dispositif selon l'une quelconque des revendications 1 à 6, **caractérisé en ce qu'**il comprend des moyens de ventilation pour créer un flux d'air qui emporte ledit brouillard (19) de gouttelettes (18) vers l'extérieur dudit dispositif (40).

8. Dispositif selon l'une quelconque des revendications 1 à 7, **caractérisé en ce qu'**il comprend un tube de collecte (7) apte et disposé à recueillir le jet de liquide (17) sortant de l'orifice de sortie (15) et à se vider dans ledit réservoir de collecte (6).

9. Dispositif selon l'une quelconque de revendications 1 à 8, **caractérisé en ce qu'**il comprend un réservoir secondaire de liquide relié au réservoir primaire (6), de préférence par l'intermédiaire d'une pompe.

10. Dispositif selon l'une quelconque des revendications 1 à 9, **caractérisé en ce qu'**il comprend un moyen de chauffage apte à évaporer le liquide résiduel dans ledit dispositif après son arrêt.

**11.** Dispositif selon l'une quelconque des revendications 1 à 10, **caractérisé en ce qu'**il comprend au moins un moyen (9, 24) de détection d'un manque de liquide associé à une boucle de rétroaction pour couper ou diminuer l'intensité des ondes acoustiques émises par l'élément piézo-électrique (1) en cas de manque d'eau.

**12.** Dispositif selon la revendication 11, **caractérisé en ce que** ledit moyen de détection d'un manque de liquide est un capteur ou une pluralité de capteurs, et/ou comprend une mesure d'un paramètre électrique de la pompe de circulation (10).

**13.** Dispositif selon la revendication 11 ou 12, **caractérisé en ce qu'**il comprend un détecteur de niveau d'eau (9) dans le réservoir primaire (6).

**14.** Procédé de mise en route d'un dispositif selon l'une quelconque des revendications 11 à 13, dans lequel

(a) On fait enter du liquide dans le réservoir primaire (6) par l'orifice d'entrée (13) ;
(b) Lorsque le niveau dudit liquide monte dans ledit réservoir primaire (6) jusqu'à un point préréglé qui est détecté par un détecteur de niveau d'eau (9) dans le réservoir primaire (6), on met en fonctionnement la pompe de circulation (10) ;
(c) La pompe de circulation (10) créé une pression de liquide suffisante pour que le liquide puisse envahir la buse (4), éventuellement après avoir envahi la chambre de mise en pression (5), et pour former un jet de liquide (17) stable qui sort de l'orifice de sortie (15), sachant que pendant au moins une partie de ce temps, on fait entrer du liquide dans le réservoir primaire (6) par l'orifice d'entrée (13) ;
(d) Lorsque le niveau dudit liquide dans ledit réservoir primaire a atteint un point préréglé qui est détecté par un détecteur de niveau, on active l'alimentation électrique de l'élément piézo-électrique (1) pour créer des gouttelettes de liquide.

**15.** Procédé selon la revendication 14, dans lequel dans l'étape (d) ledit point préréglé et/ou ledit détecteur de niveau son le(s) même(s) qu'à l'étape (b).

**Patentansprüche**

**1.** Zerstäubungsvorrichtung (40), umfassend

a) eine Zerstäubungsdüse (4), versehen mit mindestens einer Öffnung (14) zur Aufnahme von Flüssigkeit und mindestens einer Öffnung zum Austritt von Flüssigkeit (15) und auf der gegenüber liegenden Seite der Öffnung zum Austritt (15) einem piezoelektrischen Element (1), das ausgelegt ist, um akustische Wellen in der Flüssigkeit zu emittieren,
und wobei der Querschnitt der Düse (4) eine fortschreitende Verengung in Richtung der ersten Öffnung zum Austritt (15) aufweist, so dass in der Düse (4) die akustischen Wellen fokalisiert sind, um einen Nebel (19) aus Tröpfchen (18) der Flüssigkeit zu erzeugen;
b) einen Sammelbehälter (6), der die Düse (4) mit Flüssigkeit versorgt;
c) eine Pumpe (10), genannt "Zirkulationspumpe", die einerseits mit dem Sammelbehälter (6) und andererseits mit der Düse (4) durch die mindestens eine Öffnung (14) zur Aufnahme verbunden ist, die in der Düse (4) angebracht ist, wobei die zirkulationspumpe (10) ausgelegt ist, um in der Düse (4) einen Flüssigkeitsdruck zu generieren, der ausreichend ist, um einen Flüssigkeitsstrahl (17) aufrechtzuerhalten, der aus der Öffnung zum Austritt (15) der Düse (4) austritt;
d) eine Druckkammer (5), die von der Flüssigkeit durchquert wird, die aus der Zirkulationspumpe (5) austritt, vor ihrem Eintritt in die Düse (4),

wobei die Zerstäubungsvorrichtung **dadurch gekennzeichnet ist, dass** sich das Volumen (V5) des oberen Teils der Druckkammer (5), der sich auf einem Flüssigkeitsniveau befindet, das sich über dem höchsten der drei folgenden Punkte befindet: der Öffnung (14) zur Aufnahme von Wasser der Düse, die sich am höchsten befindet, dem oberen Rand der Öffnung (15) zum Austritt der Düse (4), dem höchsten Punkt des piezoelektrischen Elements (1), mindestens zweimal (vorzugsweise mindestens sechsmal und noch bevorzugter mindestens zwölfmal) größer als das Volumen (V4) der Düse (4) ist.

**2.** Vorrichtung nach Anspruch 1, **dadurch gekennzeichnet, dass** der Querschnitt der Düse (4) eine derartige Verengung aufweist, dass die akustischen Wellen auf dem Niveau der Öffnung zum Austritt (15) fokalisiert sind.

**3.** Vorrichtung nach Anspruch 1 oder 2, **dadurch gekennzeichnet, dass** die Summe der Oberflächen der Öffnungen zur Aufnahme (14) größer und vorzugsweise mindestens dreimal größer als der Abschnitt der Öffnung zum Austritt (15) ist.

**4.** Vorrichtung nach einem der Ansprüche 1 bis 3, **dadurch gekennzeichnet, dass** die Längsachse der Düse (4) einen Neigungswinkel $\alpha$ mit Bezug auf die Horizontale bildet, der zwischen 0° und 45º, vorzugsweise zwischen 0° und 30° und noch bevorzugter zwischen 5º und 20º liegt.

**5.** Vorrichtung nach einem der Ansprüche 1 bis 4, **dadurch gekennzeichnet, dass** sie mindestens eine Platte zur Stabilisierung (16) des Flüssigkeitsniveaus umfasst, die horizontal, vertikal und schräg angeordnet ist, umfassend jeweils mindestens eine Öffnung (20) und/oder bildend mindestens eine Öffnung (20) mit mindestens einer weiteren Platte zur Stabilisierung (16) oder einer Wand des Behälters (6).

**6.** Vorrichtung nach Anspruch 5, **dadurch gekennzeichnet, dass** die Platten zur Stabilisierung (16) mindestens zwei an der Zahl und derart angeordnet sind, dass die Öffnungen (20) mit Bezug aufeinander versetzt sind.

**7.** Vorrichtung nach einem der Ansprüche 1 bis 6, **dadurch gekennzeichnet, dass** sie Ventilationsmittel umfasst, um einen Luftfluss zu erzeugen, der den Nebel (19) aus Tröpfchen (18) an das Äußere der Vorrichtung (40) trägt.

**8.** Vorrichtung nach einem der Ansprüche 1 bis 7, **dadurch gekennzeichnet, dass** sie ein Sammelrohr (7) umfasst, das ausgelegt und angeordnet ist, um den Flüssigkeitsstrahl (17) aufzufangen, der aus der Öffnung zum Austritt (15) austritt, und sich in den Sammelbehälter (6) zu leeren.

**9.** Vorrichtung nach einem der Ansprüche 1 bis 8, **dadurch gekennzeichnet, dass** sie einen sekundären Flüssigkeitsbehälter umfasst, der mit dem primären Behälter (6) verbunden ist, vorzugsweise mit Hilfe einer Pumpe.

**10.** Vorrichtung nach einem der Ansprüche 1 bis 9, **dadurch gekennzeichnet, dass** sie ein Heizmittel umfasst, das ausgelegt ist, um die Restflüssigkeit in der Vorrichtung nach ihrem Stopp zu verdampfen.

**11.** Vorrichtung nach einem der Ansprüche 1 bis 10, **dadurch gekennzeichnet, dass** sie mindestens ein Mittel (9, 24) zum Nachweis eines Fehlens von Flüssigkeit umfasst, verbunden mit einer Rückkopplungsschleife, um die akustischen Wellen zu unterbrechen oder ihre Intensität zu verringern, die vom piezoelektrischen Element (1) im Fall des Fehlens von Wasser emittiert werden.

**12.** Vorrichtung nach Anspruch 11, **dadurch gekennzeichnet, dass** das Mittel zum Nachweis eines Fehlens von Flüssigkeit ein Sensor oder eine Vielzahl von Sensoren ist und/oder eine Messung eines elektrischen Parameters der Zirkulationspumpe (10) umfasst.

**13.** Vorrichtung nach Anspruch 11 oder 12, **dadurch gekennzeichnet, dass** sie einen Detektor des Wasserniveaus (9) im primären Behälter (6) umfasst.

**14.** Verfahren zur Inbetriebnahme einer Vorrichtung nach einem der Ansprüche 11 bis 13, wobei

(a) Flüssigkeit in den primären Behälter (6) durch die Öffnung (13) zum Eintritt eingelassen wird;
(b) wenn das Niveau der Flüssigkeit in dem primären Behälter (6) bis auf einen voreingestellten Punkt ansteigt, der von einem Detektor des Wasserniveaus (9) im primären Behälter (6) nachgewiesen wird, die Zirkulationspumpe (10) in Betrieb gesetzt wird;
(c) die Zirkulationspumpe (10) einen Flüssigkeitsdruck erzeugt, der ausreichend ist, damit die Flüssigkeit in die Düse (4) eindringen kann, eventuell, nachdem sie in die Druckkammer (5) eingedrungen ist, und um einen stabilen Flüssigkeitsstrahl (17) zu bilden, der aus der Öffnung zum Austritt (15) austritt, wobei während mindestens eines Teils dieser Zeit Flüssigkeit in den primären Behälter (6) durch die Öffnung zum Eingang (13) eingelassen wird;
(d) wenn das Niveau der Flüssigkeit in dem primären Behälter bis auf einen voreingestellten Punkt gestiegen ist, der von einem Detektor des Wasserniveaus nachgewiesen wird, die elektrische Versorgung des piezoelektrischen Elements (1) aktiviert wird, um Flüssigkeitströpfchen zu erzeugen.

**15.** Verfahren nach Anspruch 14, wobei in Schritt d) der voreingestellte Punkt und/oder der Niveaudetektor der/die gleiche(n) wie in Schritt (b) ist/sind.

**Claims**

**1.** Nebuliser device (40) comprising

a) a nebuliser nozzle (4) provided with at least one opening (14) for the intake of liquid and with at least one opening for the outlet of liquid (15), and on the side opposite said outlet opening (15) a piezoelectric component (1) which can emit acoustic waves into said liquid,
and the cross-section of said nozzle (4) having a progressive narrowing in the direction of said first outlet opening (15), in such a way that in said nozzle (4) the acoustic waves are focussed in order to create a mist (19) of droplets (18) of said liquid;
b) a collection reservoir (6) which supplies said nozzle (4) with liquid;
c) a pump (10) referred to as "circulation pump" connected on the one hand to the collection reservoir (6) and on the other hand to said nozzle (4) by the at least one intake opening (14) arranged in said nozzle (4), said circulation pump (10) being able to generate in said nozzle (4) a liquid pressure sufficient to maintain a stream of liquid (17) emerging via said outlet opening (15) of the nozzle (4);
d) a pressurising chamber (5) through which passes the liquid emerging from the circulation pump (5) before entering said nozzle (4),

said nebuliser device being **characterised in that** the volume (V5) of the upper portion of the pressurising chamber (5) located at a liquid level which is higher than the highest of the following three points: the highest opening (14) for the intake of water of the nozzle, the upper edge of the outlet opening (15) of the nozzle (4), the highest point of the piezoelectric component (1), is at least twice (preferably at least six times and more preferable at least twelve times) the volume (V4) of the nozzle (4),

**2.** Device according to claim 1, **characterised in that** the cross-section of said nozzle (4) has a narrowing such that the acoustic waves are focussed on the level of said outlet opening (15).

**3.** Device according to claim 1 or 2, **characterised in that** the sum of the surfaces of the intake openings (14) is greater, and preferably at least three times greater, than the section of the outlet opening (15).

**4.** Device according to any of claims 1 to 3, **characterised in that** the longitudinal axis of said nozzle (4) forms an angle of inclination $\alpha$ with respect to the horizontal which is between 0° and 45°, preferably between 0° and 30° and more preferably between 5° and 20°.

**5.** Device according to any of claims 1 to 4, **characterised in that** it comprises at least one stabilisation plate (16) of the liquid level, arranged horizontally, vertically or on a slant, each comprising at least one opening (20) and/or forming at least one opening (20) with at least one other stabilisation plate (16) or a wall of said reservoir (6).

**6.** Device according to claim 5, **characterised in that** said stabilisation plates (16) number at least two and are arranged in such a way that the openings (20) are offset from one another.

**7.** Device according to any of claims 1 to 6, **characterised in that** it comprises means of ventilation for creating a flow of air that carries away said mist (19) of droplets (18) to the outside of said device (40).

**8.** Device according to any of claims 1 to 7, **characterised in that** it comprises a collection tube (7) able and arranged to collect the stream of liquid (17) emerging from the outlet opening (15) and to empty into said collection reservoir (6).

**9.** Device according to any one of claims 1 to 8, **characterised in that** it comprises a secondary reservoir of liquid connected to the primary reservoir (6), preferably by the intermediary of a pump.

**10.** Device according to any of claims 1 to 9, **characterised in that** it comprises a means of heating able to evaporate the residual liquid in said device after the stopping thereof.

**11.** Device according to any of claims 1 to 10, **characterised in that** it comprises at least one means (9, 24) for detecting a lack of liquid associated with a feedback loop in order to cut off or reduce the intensity of the acoustic waves emitted by the piezoelectric component (1) in the event of a lack of water.

12. Device according to claim 11, **characterised in that** said means for detecting a lack of liquid is a sensor or a plurality of sensors, and/or comprises a measurement of an electrical parameter of the circulation pump (10).

13. Device according to claim 11 or 12, **characterised in that** it comprises a water level detector (9) in the primary reservoir (6).

14. Method for turning on a device according to any of claims 11 to 13, wherein

(a) Liquid is made to enter into the primary reservoir (6) through the intake opening (13);
(b) When the level of said liquid rises in said primary reservoir (6) to a preset point which is detected by a water level detector (9) in the primary reservoir (6), the circulation pump (10) is turned on;
(c) The circulation pump (10) creates a liquid pressure that is sufficient for the liquid to invade the nozzle (4), optionally after having invaded the pressurising chamber (5), and to form a stable stream of liquid (17) that emerges from the outlet opening (15), knowing that during at least one portion of this time, liquid is made to enter the primary reservoir (6) through the intake opening (13);
(d) When the level of said liquid in said primary reservoir has reached a preset point which is detected by a level detector, the electrical power of the piezoelectric component (1) is activated in order to create droplets of liquid.

15. Method according to claim 14, wherein in the step (d) said preset point and/or said level detector are the same as in the step (b).

Figure 1a

Figure 1b

Figure 2a

Figure 2b

Figure 2c

Figure 2d

Figure 3a

Figure 3b

Figure 4a

Figure 4b

Figure 4c

Figure 4d

Figure 5a

Figure 5b

**EP 2 991 773 B1**